# EUROPEAN PATENT APPLICATION

(11) **EP 1 764 130 A1**
(43) Date of publication of application: **21.03.2007**
(21) Application number: 05108541.3
(22) Date of filing: 16.09.2005
(51) Int. Cl.: A61M 39/18

(54) **Multiple-use sterile connector**

(71) Applicant: The Automation Partnership (Cambridge) Limited, Royston, Hertfordshire SG8 5WY (GB)
(72) Inventor: Malinge, David S., Royston, Hertfordshire SG8 9DE (GB)
(74) Representative: Brunner, Michael John

(57) **Abstract**

A connector (20) has an annular body (200) with a first end (202) around port (208) and which includes an end face (203) for connection to the end face (103) of a second connector (10). The end face (203) has a face seal (204) and a membrane seal (205) closing the face seal and the port (208). A connection member (210) within the connector has a tubular spigot (211) defining a bore (213) which forms a first flow path to the port (208) and which is slidably and sealingly disposed within the annular body (200). The first end (212) of the tubular spigot is capable of movement into a bore (100) of the other connector (10) to provide a flow path between the connectors after the membrane seal (205) has been removed. At a second end (222) of the connection member, a counter-bore (230) is provided aligned with the bore (213) and forms a second port (228). An end face (223) around the second port contains a face seal (224), and a membrane seal (225) closes the face seal and second port. Adjacent the second end, a radial passage (238) opens into the counter-bore (230), forming a third flow path though which flow can be provided to the first flow path. An alternative flow path is through the second port (228) after removal of the membrane seal (225) and attachment of a further identical connector (20).

## Description

The present invention relates to multiple-use sterile connectors for use in, for example, drug-discovery processes.

Connectors of this type allow for the straightforward dry connection of two separate pre-sterilised fluid pathways, while maintaining the sterile integrity of both. Particular applications are in the transfer of inocula to reactors, the connection of sterile fluid supplies to equipment such as bio-reactors, and the connection of disposable systems which contain, for example, capsule filters or bags of sterile fluid.

One particular system, produced by Pall Corporation and known as Kleenpack^{™} connectors utilises removable sealing strips to protect the ports in male and female connectors to maintain the sterility of the sterile fluid pathway.

Whilst such connectors provide the required sterile pathway, they do not allow the connection of plural fluid sources, for example, time-after-time in a sterile way such, once a connection has been made between the male and female connectors, any disconnection of it removes the sterility, so that the attachment of a new bag or the like cannot be ensured to be sterile.

There is a need therefore to provide a connector which can be re-used, but without loss of the sterile pathway.

According to the present invention there is provided a multiple-use sterile connector having:
first, second and third fluid pathways therethrough, the second and third pathways connecting with the first pathway, and each pathway providing a port for fluid flow in to or out of the connector in use;
a first end around the first pathway port and including an end face for connection to the end face of a second connector in use, the end face of the first end having a face seal and a membrane seal closing the face seal and the port;
a second end around the second pathway port and including an end face for connection to the end face of a third connector in use, the end face of the second end having a face seal and a membrane seal closing the face seal and the port.

The third pathway may form a spigot for connection to connecting tube or the like and may be selectively closable.

The spigot may have an internal end face with an end seal selectively engageable with a valve seat in the third pathway.

Preferably, the connector has an annular body including the first end; and
a connection member having a tubular spigot defining a bore forming said first pathway and port and being slidably and sealingly disposed within the annular body for axial movement of a first end of the tubular spigot in use into a bore of the other connector,
at a second end, a counter-bore aligned with the bore and forming the second pathway and port, an end face around the second port and containing the face seal, and membrane seal closing the face seal and second port, and,
adjacent the second end, a radial passage into the counter-bore, the radial passage forming the third pathway.

The radial passage may connect with a second tubular spigot for attachment to a connecting tube or the like.

Preferably, the connector includes a sleeve member forming part of the third pathway and having a first end for attachment to a connecting tube or the like, and at a second end, a cylindrical wall slidably and sealingly engaged with the annular body and enclosing and engaging the second end of the connection member to cause, in use, the first end of the tubular spigot to be moved into the bore of the other connector, and a passage within the sleeve member around or through the second end of the connection member to allow fluid flow from first end of the sleeve member past the second end of the connection member into the counter-bore and bore of the spigot through the radial passage, further axial movement of the sleeve member along the annular body in use causing the end of the tubular spigot to be inserted further into the bore of the other connector and closing the radial passage in the tubular spigot to prevent fluid flow from the first end of the sleeve member into the counter-bore and bore, the sleeve member being arranged to be removable from the annular body after the tubular spigot has been inserted further into the bore of the other connector.

The membrane seals may be removable or pierceable.

An annular seal, which may be an O-ring seal, may be disposed in the bore of the female connector.

Three examples of connector assemblies and components according to the present invention will now be described with reference to the accompanying drawings in which:
Figs. 1A -1E illustrate a first connector assembly at different stages of use, substantially in longitudinal section;
Figs 2A - 2C illustrate a second connector assembly at different stages of use, again in longitudinal cross-section; and,
Figs 3A and 3B show a third connector assembly at different stages of use, again in longitudinal cross-section.

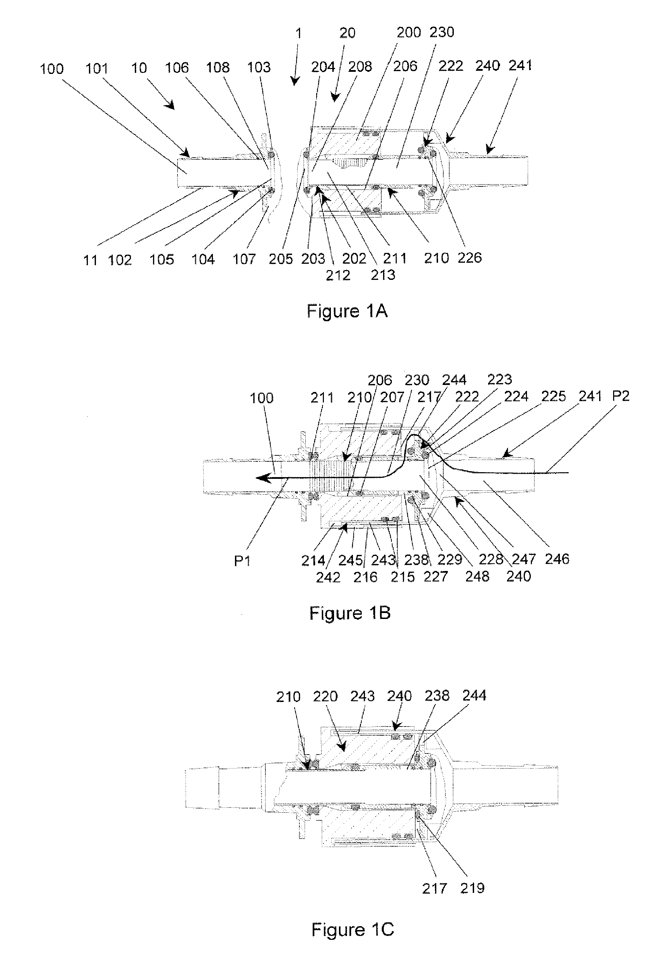

The sterile connector assembly 1 shown in Figs. 1a to 1e includes a female connector 10 and a male connector 20 which can be connected together to provide a sterile fluid path from one end of the connector to the other.

Fig. 1a shows the female connector 10 and the male connector 20 aligned, but before connection. The female connector 10 has a tubular spigot 11, which has a bore 100, and provides for connection to a flexible tube or the like (not shown) and which has a first end 101 over which the tube fits. The female connector 10 has a second end 102 for engagement with the male connector 20, the second end having an end face 103 which has a port 108, a flexible face seal 104 over which is provided a removal membrane seal 105 which closes the port and the face seal but which can be peeled away from the face seal transversely to the longitudinal axis of the female connector 10 to open the port 108. A flange 107 extends generally radially outwardly at the end 102. Disposed within the bore 100 are a pair of O-ring seals 106, the purpose of which will be described below.

The male connector 20 has an annular body 200 which has a first end 202 providing a port 208 in an end face 203 for engagement with the end face 103 of the female connector 10 by means of a face seal 204 (of the same type as the face seal 104) and which also has a removable membrane seal 205 closing the face seal 204 and the port 208 and which is peelable away from it to open the port 208 and hence the path between the connectors 10, 20 in use. Disposed through a bore 206 in the centre of the annular body 200 is a connection member 210 having a tubular spigot 211 which is capable of sliding through the bore 206 in the annular body and seals within the bore by means of a flexible ring seal, in this example, an O-ring seal 207.

Figs. 1A to 1E, for simplicity, do not show the way in which the male and female connectors are held in engagement, but cooperating axially extending flexible barbed arms on one connector can engage with appropriately formed shoulders on the other one to secure the connection.

Thus, in Fig. 1B the male and female connectors are shown connected.

Fig. 1B also shows the tubular spigot 211 of the connection member 210 inserted through the port 108 into the bore 100 and sealed against the O-ring seals 106. In order to achieve this movement the tubular spigot 211 of the connection member 210 moves through the bore 206 and the port 208 in the annular body 200. Movement of the connection member 210 is achieved by engagement of a second end 222 of the connection member 210 with a sleeve member 240 which is attached to the annular body 200 and which can slide along it.

At the second end 222 of the connection member 210, a counterbore 230 is formed which runs into a bore 213 provided within the first end 212 of the connection member. Also at the second end 222, an end face 223 is provided which has a port 228 at the end of the counterbore. The end face also has a face seal 224 identical to that of the face seal 104 of the female connector 10, and a removable membrane seal 225 closing the port 228 and the face seal 224. Within the port 228 at the end of the counterbore 230 a pair of O-ring seals 226 are provided, the internal diameter of the counterbore 230 and that of the bore 100 in the female connector 10 being identical.

The first end 212 of the connection member 210 is of reduced diameter externally, in comparison with the external diameter of the spigot at the other end 222. The end 222 has a flange 227 which is substantially identical to the flange 107, but which, additionally, has a port in the form of a notch or aperture 244 through it. On the side of the flange 227 opposite the face seal 224 another O-ring seal 229 is provided and one or more radial bores 238 extend through the wall of the spigot 212 adjacent the seal 229.

The sleeve member 240 has a first end 242 with a cylindrical wall 243 which is arranged to slide within an annular groove 214 formed in the annular body 200. A pair of O-ring seals 215 seal against the interior of the cylindrical wall to provide a fluid type connection. At the other end, a tubular spigot 241, similar to that, 101 of the female connector 10 is formed for connection to suitable flexible tubing or the like again. A bore 246 extends through the spigot 241 and opens into a enlarged diameter cavity 247. Within the cavity 247, spaced around the internal periphery of the sleeve member 240 a number of abutments 248 are provided which engage with the flange 227 on the end of the connection member 210 so that the sleeve, as it is pushed into the annular body 200 also pushes the spigot 211 of the connection member 210 into the bore 100 of the female connector 10, producing a sterile fluid seal as shown in Fig. 1B.

To provide a first stop or limit on the extent of travel of the sleeve member 240 into the annular groove in the annular body 200, a number of frangible stops 245 are provided around the periphery of the sleeve 240 and slide within slots 216 formed in the outer wall of the annular body 200. The first end position of the sleeve 240 within the annular body 200 is shown in Fig. 1B.

In this position a fluid flow pathway (P2,P1) exists, firstly (P2) from within the bore 246 of the spigot 241, into the chamber 247, through the passages or apertures 244, via the radial passages 238, then (P1) into the counter bore 230, through the bore 213 and into the bore 100 in the female connector 10.

To attach a further connector, identical to the male connector 20, for example to supply a second or replacement fluid to apparatus connected to the female connector 10, the frangible stops 245 are first broken off by twisting the sleeve member 240 about its longitudinal axis and the cylindrical wall 243 of the sleeve member 240 is then pushed further into the annular body 200. As it moves further into the annular body it pushes the connection member 210 further into the annular body 200 until the flow path through the passages 244 and 238 is closed by engagement of the seal 229 at the rear of the flange 247 against the end 217 of the annular body 200. In this position the connection member 210 is secured against the end of the annular body 200 by a suitable snap-fit coupling or the like (not shown). The position shown in Fig. 1C is thus achieved.

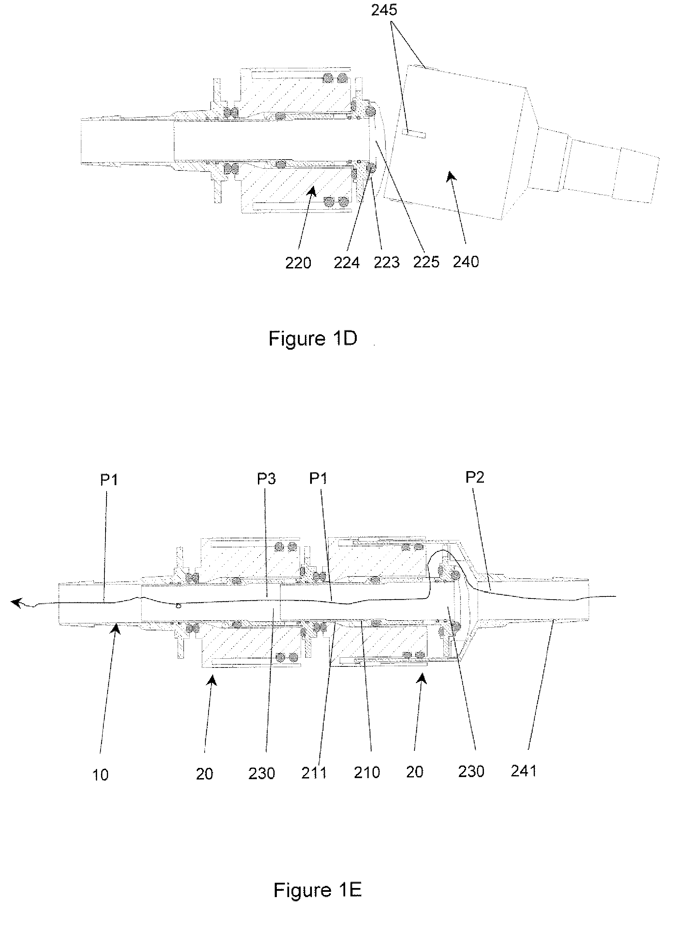

The sleeve member 240 can then be removed as shown diagrammatically in Fig. 1D, following which, because the face seal 224 and removable membrane seal 225 remain in tact, the end of the connector remains in a sterile condition so that a second male connector 20 can be attached and secured to the end face 223 in the precisely the same manner as the first male connector was attached to the female connector 10 (Figs. 1A and 1B). The membrane seals 225, 205 (on the additional male connector 20) are then removed, the spigot 211 of the connection member 210 on the additional male connector is moved into the bore 230 on the first male connector 20 and a fluid pathway (P2,P1 ,P3,P1) is opened between the additional male connector 20 and the female connector 10 (see Fig.1E).

It will be appreciated, that further disconnections and connections can be made with additional male connector members 20 in a similar manner without the sterility of the system being compromised.

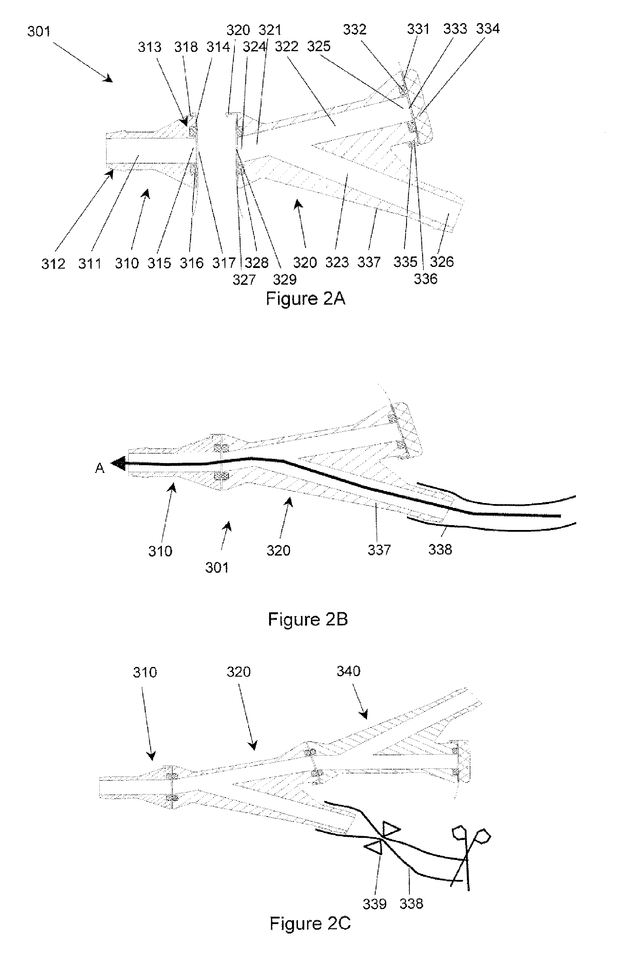

Figures 2A and 2B show a further sterile connector assembly 301 with two connectors, 310, 320.

The connector 310 has a bore 311, and provides for connection to a flexible tube or the like (not shown) leading to equipment (also not shown) and which has a first end 312 over which the tube fits. The first connector 310 has a second end 313 for engagement with the second connector 320, the second end having an end face 314 which has a port 315, a flexible face seal 316 over which is provided a removal membrane seal 317 which closes the port and the face seal but which can be peeled away from the face seal transversely to the longitudinal axis of the connector 310 to open the port 315. The rear face of the end 314 provides a shoulder 318 for a snap-fit connection to the second connector 320 (described below).

The second connector 320 has first, second and third interconnected bores 321, 322, 323 with respective ports 324, 325, 326. The bores 321 and 322 are aligned with one another. Around the first port 324, in the end face 327 is provided an end seal 328 and a removable membrane seal 329, for sealing, in use, with the face 314 at the second end 313 of the first connector 310. One of a number of snap-fit hooks 330 is shown extending forward from the end face 327 for securing the connection between the connectors 310, 320 (see Figure 2B). After connection of the connectors 31, 320, the membrane seals 317, 329 are pulled away sideways to open the pathway between the connectors.

Around the second port 325, in the corresponding end face 331, is provided an end seal 332 and a removable membrane seal 333, for sealing, in use, with the end 312 of an identical second connector 340 in use (as will be described in connection with Figure 2B below). The second port 325 is normally closed by a cap 334 which also has snap-fit hooks 335 to secure the cap, the snap-fit hooks 335 attaching to shoulders 336. The end face 331 is identical to the end face 314 of the first connector 310 to allow connection of the identical second connector 340.

The third bore 323 extends through a spigot 337 which enables connection of the third bore 323 (providing the third pathway P3) to a flexible tube 338 or the like for the supply of fluid along the path (shown by arrow A) to the first bore 321 (providing the first pathway P1) and hence to the bore 311 in the first connector 310, and from there to equipment (not shown).

When it is desired to supply fresh fluid or another fluid, the tube 338 is pinched off by a suitable clamp (indicated at 339), the tube 338 can then be cut off, and then the cap 334 is removed and the identical connector 340 is attached as shown in Figure 2C, the membrane seals 333, 329 are removed to open the fluid pathway from the connector 340 to the connector 320 (through the bore 322) and hence through the bore 321 to the first connector 310. The sterility of the connections is maintained at all times as will be appreciated from the figures and the description above.

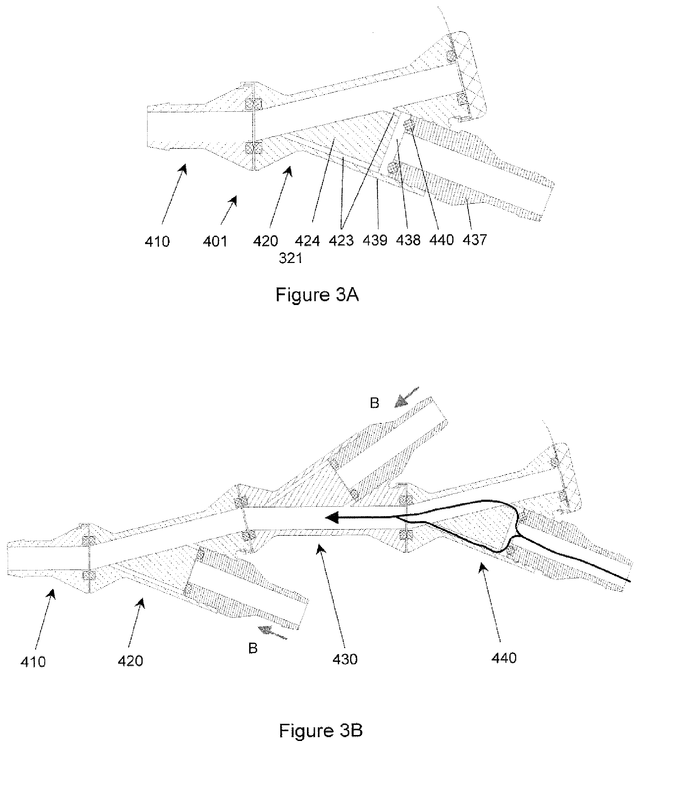

Figures 3A and 3B illustrate a further sterile connector assembly 401 with two connectors, 410, 420. The connector 410 is identical to the connector 310 shown in Figures 2A - 2C and will not be described further therefore.

The connector 420 is substantially similar to the connector 320 shown in Figures 2A - 2C and identical parts will not be further described. However the spigot 437 is longitudinally movable within a bore 438 in a socket 439 which defines plural bores 423 around a central core 424, and in the position shown in Figure 3A allows fluid flow into the connector 420. To close flow through the spigot 437, it is moved (as shown by arrow B) towards the core 424 and an end face seal 440 closes against the end face 425 of the core 424. A snap-fit (not shown) secures the position of the spigot 437 against the core 424 and the closure of the pathway into the bore 321, after which further identical connectors 430, 440 can be attached in the same way, in turn.

## Claims

1. A multiple-use sterile connector (20) having:
first (P1), second (P2) and third (P3) fluid pathways therethrough, the second and third pathways connecting with the first pathway, and each pathway providing a port (208, 228, 248) for fluid flow in to or out of the connector in use;
a first end (202) around the first pathway port (208) and including an end face (203) for connection to the end face (103) of a second connector (10) in use, the end face of the first end having a face seal (204) and a membrane seal (205) closing the face seal and the port (208);
a second end (222) around the second pathway port (228) and including an end face (223) for connection to the end face of a third connector (20) in use, the end face of the second end having a face seal (224) and a membrane seal (225) closing the face seal and the port (228).

2. A connector according to claim 1, wherein the third pathway forms a spigot for connection to connecting tube or the like.

3. A connector according to claim 1 or claim 2, wherein the third pathway is selectively closable.

4. A connector according to claim 2 and claim 3, wherein the spigot has an internal end face with an end seal selectively engageable with a valve seat in the third pathway.

5. A connector (20) according to claim 1, having
an annular body (200) including the first end (202); and
a connection member (210) having
a tubular spigot (211) defining a bore (213) forming said first pathway and port and being slidably and sealingly disposed within the annular body (200) for axial movement of a first end (212) of the tubular spigot in use into a bore (100) of the other connector,
at a second end (222), a counter-bore (230) aligned with the bore (213) and forming the second pathway and port, an end face (223) around the second port and containing the face seal (224), and membrane seal (225) closing the face seal and second port, and,
adjacent the second end, a radial passage (238) into the counter-bore (230), the radial passage forming the third pathway.

6. A connector (20) according to claim 5, wherein the radial passage (238) connects with a second tubular spigot for attachment to a connecting tube or the like.

7. A connector (20) according to claim 5, further including
a sleeve member (240) forming part of the third pathway and having a first end (241) for attachment to a connecting tube or the like, and at a second end (242), a cylindrical wall (243) slidably and sealingly engaged with the annular body (200) and enclosing and engaging the second end (222) of the connection member to cause, in use, the first end (212) of the tubular spigot (211) to be moved into the bore (100) of the other connector, and a passage (244) within the sleeve member around or through the second end (222) of the connection member to allow fluid flow from first end (241) of the sleeve member past the second end (222) of the connection member into the counter-bore (230) and bore (213) of the spigot (211) through the radial passage (238), further axial movement of the sleeve member (240) along the annular body (200) in use causing the end (212) of the tubular spigot (211) to be inserted further into the bore (100) of the other connector (10) and closing the radial passage (238) in the tubular spigot to prevent fluid flow from the first end (241) of the sleeve member into the counter-bore (230) and bore (213), the sleeve member (240) being arranged to be removable from the annular body (200) after the tubular spigot (211) has been inserted further into the bore (100) of the other connector (10).

8. A connector (20) according to any of claims 5 to 7, wherein one or more of the membrane seals (205,225) is removable.

9. A connector (20) according to any of claims 5 to 7, wherein one or more of the membrane seals (205,225) is pierceable.

10. A connector (20) according to any of claims 5 to 9, further including an annular seal (226) disposed in the counter-bore (230).

11. A connector (20) according to claim 10, wherein the annular seal (226) is an O-ring seal.

12. A connector (20) according to any of claims 5 to 11, further including a frangible stop (245) on the sleeve member (240) for defining a first limit on the extent of movement of the sleeve member and tubular spigot, the frangible stop being breakable to allow the further axial movement of the sleeve member (240) along the annular body (200) to cause the end (212) of the tubular spigot (211) to be inserted further into the bore (100) of the other connector (10) and closing the radial passage (238) in the tubular spigot to prevent fluid flow from the first end (241) of the sleeve member into the counter-bore (230) and bore (213).

13. A multiple-use sterile connector assembly (1) including a connector according to any of claims 5 to 12, further comprising:
a female connector (10) having
a bore (100);
a first end (101) for attachment to a connecting tube or the like; and
a second, engagement, end (102) having an end face (103) containing a face seal (104), and a membrane seal (105) closing the face seal.

14. A multiple-use sterile connector assembly (1) according to claim 13, wherein membrane seal (105) in the female connector (10) is removable.

15. A multiple-use sterile connector assembly (1) according to claim 13, wherein membrane seal (105) in the female connector (10) is pierceable.

16. A multiple-use sterile connector assembly (1) according to any of claims 13 to 15, further including an annular seal (106) disposed in the bore (100) of the female connector (10).

17. A multiple-use sterile connector assembly (1) according to claim 16, wherein the annular seal (106) is an O-ring seal.
